# EUROPEAN PATENT SPECIFICATION

(11) **EP 1 463 440 B1**
(45) Date of publication and mention of the grant of the patent: **02.12.2009**
(21) Application number: 02804687.8
(22) Date of filing: 02.12.2002
(51) Int. Cl.: A61B 1/00, A61K 31/04, A61K 33/00, A61K 45/06, A61P 9/08

(54) **PREVENTION OF FLAP NECROSIS IN PLASTIC SURGERY**
VERHINDERUNG VON HAUTLAPPENNEKROSE IN DER PLASTISCHEN CHIRURGIE
PREVENTION DE LA NECROSE DES LAMBEAUX EN CHIRURGIE PLASTIQUE

(30) Priority: 06.12.2001 US 336175 P
(43) Date of publication of application: 06.10.2004
(73) Proprietor: Duke University, Durham, NC 27708-0083 (US)
(72) Inventor: STAMLER, Jonathan, S., Chapel Hill, NC 27514 (US); ZENN, Michael, R., Chapel Hill, NC 27516 (US); TOONE, Eric, J., Durham, NC 27705 (US)
(74) Representative: Crump, Julian Richard John
(86) International application number: PCT/US2002/036138
(87) International publication number: WO 2003/049593

(56) References cited:
- EP-A- 1 051 972
- WO-A-00/54773
- WO-A-98/08482
- US-B1- 6 359 182
- US-B2- 6 403 759
- DATABASE CAPLUS [Online] KIM ET AL.: 'Nitric oxide prevents the bovine cerebral endothelial cell death induced by serum-deprivation', XP002970750 Retrieved from STN Database accession no. 1997:786946 & KOREAN JOURNAL OF PHYSIOLOGY & PHARMACOLOGY vol. 1, no. 5, 1997, pages 515 - 521
- DATABASE CAPLUS [Online] FARINELLI ET AL.: 'Nitric oxide delays the death of trophic factor-derived PC12 cells and sympathetic neurons by a cGMP-mediated mechanism', XP002970751 Retrieved from STN Database accession no. 1996:184698 & JOURNAL OF NEUROSCIENCE vol. 16, no. 7, 1996, pages 2325 - 2334

## Description

### Technical Field

This invention is directed to a method for resolving or preventing vasoconstriction and preventing depletion of or restoring blood flow in a pedicle flap.

### Background of the Invention

In plastic/microvascular surgery for providing new tissue where such is necessary because of traumatic or other injury or for reconstruction after surgery to remove cancerous and surrounding tissue, a flap may be used for reconstruction. Specifically, a flap is a block of tissue isolated on its nutrient blood supply. Any time tissue is transferred on its pedicle (artery and vein), it is subject to vasospasm and thrombosis which may lead to necrosis of the tissue, if uncorrected.

In an attempt to prevent flap necrosis, it is standard procedure to topically apply a vasodilator such as lidocaine or paparavine to pedicle which attaches the pedicle flap to its source of blood supply. This resolves or prevents vasoconstriction intraoperatively but that effect is not always effective for preventing depletion of or restoring blood flow. Moreover, this effect is sliort-lived, decreasing to about 50% or less of baseline 30 minutes after lidocaine application.

### Summary of the Invention

It has been found herein that the effect of lidocaine in resolving or preventing vasoconstriction and preventing depletion of or restoring blood flow in a pedicle flap can be improved and lengthened by use of nitric oxide (NO) and/or NO donor that causes formation of nitrosothiol in tissue as the only vasodilator or in combination with lidocaine compared to conventional use of lidocaine alone.

The invention is directed to a method for preventing necrosis in a pedicle flap comprising topically applying to pedicle or other source of blood supply a therapeutically effective amount of vasodilator composition containing NO or NO donor or prodrug that causes formation of nitrosothiol in tissue optionally in combination with lidocaine.

The term "therapeutically effective amount" is used herein, in respect to the first embodiment, to mean a vasoconstriction resolving or preventing amount and blood flow depletion preventing or restoring amount.

### Brief Description of the Drawing

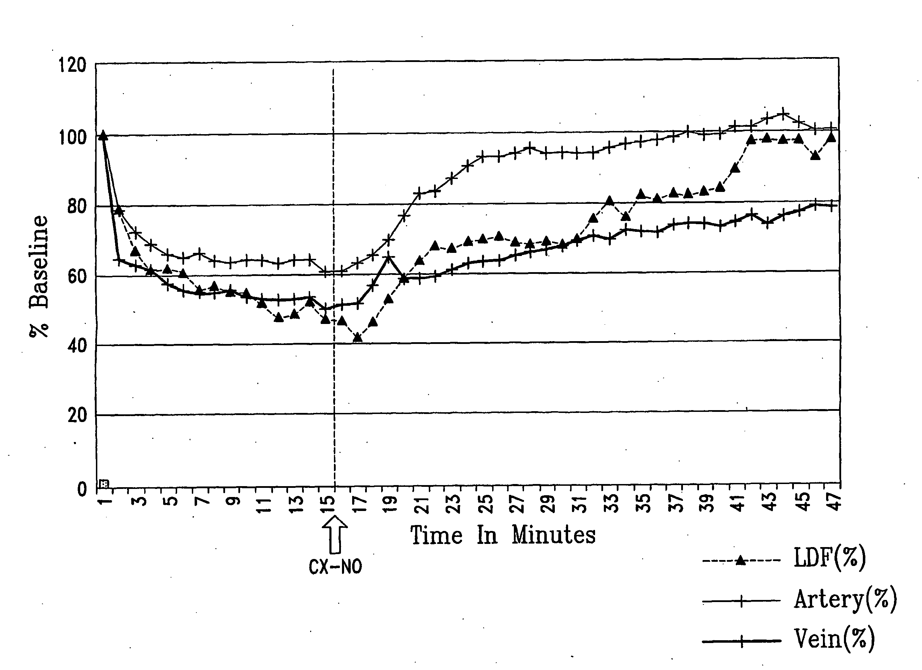

FIG. 1 is a graph of time in minutes versus % baseline and sets for the results of Background Example 2.

### Detailed Description

The invention provides a method for preventing necrosis in a pedicle flap comprising topically applying to the pedicle a therapeutically effective amount ofvasodilation composition containing NO or NO donor that causes formation ofnitrosothiol in tissue optionally in combination with lidocaine.

The NO, NO donors, that cause formation of nitrosothiol in tissue, are topically applied in compositions in the invention.

Nitric oxide is readily applied as a water solution.

A NO donor is administered. An NO donor donates nitric oxide or a related redox species and more generally provides nitric oxide bioactivity, that is activity which is identified with nitric oxide, e.g., vasorelaxation or stimulation or inhibition of a receptor protein, e.g., ras protein, adrenergic receptor, NFκB. NO donors including S-nitroso, O-nitroso, C-nitroso and N-nitroso compounds and nitro derivatives thereof and metal NO complexes, but not excluding other NO bioactivity generating compounds, useful herein, are described in "Methods in Nitric Oxide Research," edited by Feelisch, M., and Stamler, J. S., John Wiley & Sons, New York, 1996, pages 71-115 which is incorporated herein by reference. NO donors which are C-nitroso compounds where nitroso is attached to a tertiary carbon which are useful herein include those described in U.S. Patent Application No. 09/695,934 which has matured into U.S. Patent No. 6,359,182 and those described in WO 02/34705. Examples of S-nitroso compounds including S-nitrosothiols useful herein include, for example, S-nitrosoglutathione, S-nitroso-N-acetylpenicillamine, S-nitroso-cysteine and ethyl ester thereof, S-nitroso cysteinyl glycine, S-nitroso-gamma-methyl-L-homocysteine, S-nitroso-L-homocysteine, S-nitroso-gamma-thio-L-leucine, S-nitroso-delta-thio-L-leucine, and S-nitrosoalbumin. An example of an S-nitrosylated or an O-and S-nitrosylated compound is nitrosylated polythiolated cyclodextrin (hereinafter cyclodextrin NO or CX-NO) as described in Stamler, et al U.S. Patent No. 6,403,759 which can be, for example O- and S- nitrosylated β-cyclodextrin as described in Example 14 of U.S. Patent No. 6,403,759 or nitrosylated perthiolated-β-cyclodextrin as described in Examples 3-6 of U.S. Patent No. 6,403,759. Examples of other NO donors useful herein are metal nitrosyls such as sodium nitroprusside (nipride), alkyl nitrites of molecular weight up to 10,000 such as ethyl nitrite, nitroglycerin, SIN1 which is molsidomine, furoxamines, N-hydroxy (N-nitrosamine), perfluorocarbons that have been saturated with NO or a hydrophobic NO donor, and NO entrained in carbon monotubules.

Examples of C-nitroso compounds that are NO donors include: 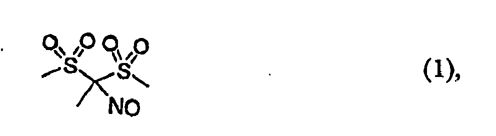 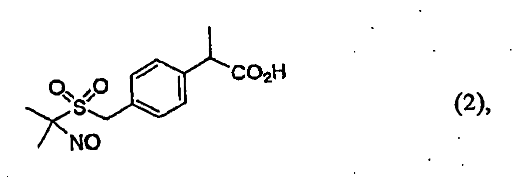 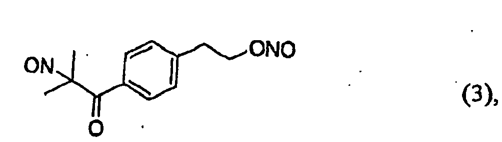 and

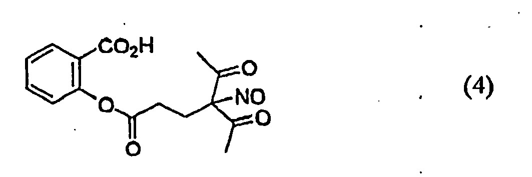

NO donors that cause formation of nitrosothiol in tissue, that are used in working examples are ethyl nitrite or nitrosylated polythiolated cyclodextrin.

As indicated above, the NO or NO donor is administered in a therapeutically effective amount. In general, these are applied at a concentration ranging from 1 µM to 100 mM, with variation within the range depending on the agent administered. Ethyl nitrite is available as 90-95% ethyl nitrite in ethanol and can be applied as an ethanol solution at a concentration, e.g., of 5X10⁻³-5X10⁻⁴ M. Cyclodextrin NO can be applied as a solution or gel or as fine particles in a pharmaceutical base at a concentration of 1 µM to 100 mM.

The NO or NO donor that causes formation of nitrosothiol in tissue is used in combination with lidocaine. The lidocaine can be applied in solution or in a cream or as viscous lidocaine present in the applied composition, e.g., at 2 to 20%.

The NO or NO donor that causes formation of nitrosothiol in tissue, and lidocaine, if used, can be topically applied in a liquid or viscous composition, e.g., as a solution, cream, ointment or gel.

The CX-NO can be formulated in a gel or solution or as fine particles in a pharmaceutical base and the drug later admixed or the drug and CX-NO can be formulated together in a gel or solution or pharmaceutical base or the drug can be incorporated before the CX-NO.

Composition containing the drug and CX-NO can be administered by application topically to the pedicle where it acts locally.

The invention is supported by the following background examples, which uses a standard model for determining flap blood flow. In the Background Example 1, ENO means ethyl nitrite. In the Background Example 2, CX-NO means nitrosylated polythiolated cyclodextrin.

### Background Example 1

Male rats (N=9) were anesthetized and skin flaps (3 x 3 cm) were raised bilaterally based on the epigastric artery and vein. Pedicle vasoconstriction was induced by topically applying 10⁻⁵ M endothenin-1 (ET-1). Fifteen minutes after ET-1 application, either 2% lidocaine or 10⁻⁴ M ENO, dissolved in saline, was applied. Over the subsequent 30 min, flap blood flow was measured with a laser Doppler flowmeter and the diameter of pedicle vessels was measured using videomicroscopy. Data are expressed as % of baseline (mean±SEM). Pairwise comparisons were performed using a Student's t-test. ET-1 caused a 69-76% vasoconstriction of the epigastric artery, 43-72% vasoconstriction of the epigastric vein, and a reduction of flap blood flow to 35-45% of baseline. Application of lidocaine caused a rapid arterial vasodilation to 101±6% of baseline within 1 min, but after 30 min the effect decreased to 82±6% of baseline. The vein dilated to 68±7% of baseline 30 min after lidocaine application. Lidocaine also caused an increase of blood flow to 87±13% of baseline in 2 min, but only 55±7% of baseline after 30 min. ENO slowly dilated the artery to 83±5% of baseline after 1 min and 98±5% of baseline after 30 min and dilated the vein to 75±5% of baseline after 30 min. ENO restored blood flow to 62±6% of baseline after 2 min and 86±10% of baseline after 30 min. ENO caused a greater restoration of blood flow and arterial diameter (p<.05) compared to lidocaine beginning 13 min (for blood flow) or 10 min (for diameter) after application of each dilator. Lidocaine was shown to have a faster effect, but ENO had a longer lasting and greater effect on arterial dilation and blood flow.

### Background Example 2

Adult male rats (six) weighing 250-250 gm were anesthetized with intraperitoneal sodium pentobartital at initial doses of 50 mg/kg after being induced with isofluorane as an inhalational anesthetic. Supplemental pentobarbital was administered as needed. The rats' core temperature was measured via rectal probe and maintained at 36-38° C with a heating pad. The groin and abdomen were shaved.

A tracheotomy was performed, and the rats intubated directly to help eliminate motion associated with respiratory movements. Bilateral 3x3 cm island skin flaps based upon the epigastic artery and vein were raised. The epigastic artery and vein were carefully isolated from each other and from surrounding soft tissue. Dissection was performed with the aid of a surgical microscope. After elevation of the flap, it was positioned on a clear acrylic sheet and maintained at its original dimensions with 4-0 nylon sutures. A laser Doppler flow probe was placed on the center of the flap. A video camera was connected to the camera port opening of the microscope. Camera output was connected to a video timer, a 13-inch video monitor, and a videotape recorder. Diameter measurements were made using a digital caliper with 100-micrometer resolution. After dissection, the rats were allowed to stabilize for 60 minutes before baseline measurements were recorded and the experiment begun in order to better help control any vascular changes associated with the preparation of the model.

Vasoconstriction was induced by adding a drop of Endothelin-1 (ET-1) at 10⁻⁵ M concentration directly to the exposed vascular pedicle using a 27 gauge needle on a tuberculin syringe. ET-1 was dissolved in 0.1% acetic acid and stored at 20° C. Data collected each subsequent minute included the arterial diameter, vein diameter, and laser Doppler flow. Fifteen (15) minutes following the administration of ET-1, 0.25ml of 3.17mM CX-NO in dimethylsulfoxide (DMSO) was applied. Data were gathered for an additional thirty (30) minutes. The rats were then sacrificed with an overdose of pentobarbital.

The results are shown in FIG. 1 where the upper curve is for arterial diameter as a percentage of baseline, the lowest curve is for vein diameter as a percentage of baseline and the dashed curve shows measurement of blood flow with a laser Doppler flowmeter and shows blood flow in small vessels as a percentage of baseline flow. The results show that the CX-NO caused increase in arterial and vein diameter and increase in blood flow in small vessels.

The invention is illustrated in the following working examples.

### Example I

A 40-year-old white male with a head and neck tumor resected and reconstructed with a free tissue transfer of forearm tissues to the face develops vasospasm after reattachement and no blood flow is seen. Topical application of ethyl nitrite (100 µM) results in vasodilation and flow is restored. The same result is obtained when compound (1) described above is applied at a concentration of 50mM instead of the ethyl nitrite. The same result is obtained when sodium nitroprusside is applied at a concentration of 50 mM instead of the ethyl nitrite.

### Example II

A 60-year-old undergoing emergent CABF (coronary artery bypass) develops cardiac ischemia after grafting, fromvasospasm of the grafts. Topical application of lidocaine (2%) resulted in temporary but marginal blood flow. Addition of 100 µM ethyl nitrite restored flow and normalized EKG (ischemia).

### Example III

CX-NO of Example 14 of U.S. Patent No. 6,403,759 is formulated in a gel at 50 mM and is applied topically to skin to relieve pain of osteoarthritis.

### Example IV

CX-NO of Example 14 of U.S. Patent No. 6,403,759 is formulated in a gel at 50 mM and is admixed with aspirin and the combination used to fill capsules each containing 100 mg aspirin and 200 mg of CX-NO. Administration of capsules orally relieved headache without any gastrointestinal bleeding. Alternatively, the CX-NO is used as or included in a coating for aspirin tablets (100 mg aspirin in a tablet), and administration orally of the coated aspirin provides the same result.

### Example V

The CX-NO of Example 14 of U.S. Patent No. 6,403,759 is formulated in a gel at 50 mM together with a therapeutic amount of insulin. Application to skin is a treatment for Type I diabetes.

### Example VI

The CX-NO of Example 14 of U.S. Patent No. 6,403,759 is formulated with hemoglobin based blood substitute in a ratio of hemoglobin to NO of 500: 1. The formulation is administered to a patient to improve peripheral blood flow and mitigate hypertension.

### Variations

Variations will be obvious to those skilled in the art. Thus, the scope of the invention is defined by the claims.

## Claims

1. A composition containing NO or an NO donor selected from the group consisting of an alkyl nitrite, a S-nitroso compound, and a metal nitrosyl compound, optionally in combination with lidocaine, for use in preventing necrosis in a pedicle flap, wherein said medicament is for topical application to the pedicle.

2. A composition according to claim 1, wherein the composition contains alkyl nitrite of molecular weight up to 10,000.

3. A composition according to claim 2, wherein the alkyl nitrite is ethyl nitrite.

4. A composition according to claim 1, where the composition contains an S-nitrosothiol.

5. A composition according to claim 4, where the S-nitrosothiol is cyclodextrin NO.

6. A composition according to claim 1, where the composition contains a metal nitrosyl.

7. Use of a composition containing NO or an NO donor selected from the group consisting of an alkyl nitrite, a S-nitroso compound and a metal nitrosyl compound, optionally in combination with lidocaine, for the manufacture of a medicament for preventing necrosis in a pedicle flap, wherein said medicament is for topical application to the pedicle.

## Patentansprüche

1. Zusammensetzung enthaltend NO oder einen NO-Donor, der ausgewählt ist aus der Gruppe, die aus einem Alkylnitrit, einer S-Nitrosoverbindung und einer Metallnitrosylverbindung besteht, optional in Kombination mit Lidocain, zur Verwendung zum Verhindern von Nekrose in einem Stiellappen, wobei das Arzneimittel zur topischen Anwendung an dem Stiel ist.

2. Zusammensetzung nach Anspruch 1, wobei die Zusammensetzung Alkylnitrit eines Molekulargewichts bis zu 10.000 enthält.

3. Zusammensetzung nach Anspruch 2, wobei das Alkylnitrit Ethylnitrit ist.

4. Zusammensetzung nach Anspruch 1, wobei die Zusammensetzung S-Nitrosothiol enthält.

5. Zusammensetzung nach Anspruch 4, wobei das S-Nitrosothiol Cyclodextrin-NO ist.

6. Zusammensetzung nach Anspruch 1, wobei die Zusammensetzung ein Metallnitrosyl enthält.

7. Verwendung einer Zusammensetzung, die NO oder einen NO-Donor enthält, der ausgewählt ist aus der Gruppe, die aus einem Alkylnitrit, einer S-Nitrosoverbindung und einer Metallnitrosylverbindung besteht, optional in Kombination mit Lidocain, zur Herstellung eines Arzneimittels zum Verhindern von Nekrose in einem Stiellappen, wobei das Arzneimittel zur topischen Anwendung an dem Stiel ist.

## Revendications

1. Composition contenant du NO ou un donneur de NO choisi dans l'ensemble constitué par un nitrite d'alkyle, un composé S-nitroso, et un composé métal nitrosyle, éventuellement en combinaison avec de la lidocaine, pour une utilisation dans la prévention contre une nécrose dans un lambeau pédiculé, ledit médicament étant destiné à être appliqué par voie topique sur le pédicule.

2. Composition selon la revendication 1, laquelle composition contient un nitrite d'alkyle ayant une masse moléculaire allant jusqu'à 10 000.

3. Composition selon la revendication 2, dans laquelle le nitrite d'alkyle est le nitrite d'éthyle.

4. Composition selon la revendication 1, laquelle composition contient un S-nitrosothiol.

5. Composition selon la revendication 4, dans laquelle le S-nitrosothiol est la cyclodextrine NO.

6. Composition selon la revendication 1, laquelle composition contient un métal nitrosyle.

7. Utilisation d'une composition contenant du NO ou un donneur de NO choisi dans l'ensemble constitué par un nitrite d'alkyle, un composé S-nitroso, et un composé métal nitrosyle, éventuellement en combinaison avec de la lidocaïne, pour la fabrication d'un médicament pour la prévention contre une nécrose dans un lambeau pédiculé, ledit médicament étant destiné à être appliqué par voie topique sur le pédicule.
